# EUROPEAN PATENT APPLICATION

(11) **EP 2 517 714 A1**
(43) Date of publication of application: **31.10.2012**
(21) Application number: 12170422.5
(22) Date of filing: 28.04.2008
(51) Int. Cl.: A61K 35/14, C07K 1/00, A23J 1/00

(54) **Recombinant vitamin K dependent proteins with high sialic acid content and methods of preparing same**

(30) Priority: 26.04.2007 US 914281 P; 10.05.2007 US 917271 P
(62) Divisional of application: 08747060.5
(71) Applicant: Inspiration Biopharmaceuticals, Inc., Laguna Niguel, CA 92677 (US)
(72) Inventor: Griffith, Michael J., San Juan Capistrano, CA 92675 (US); Drohan, Marian, Germantown, MD 20876 (US)
(74) Representative: Pallard, Caroline Chantal Patricia

(57) **Abstract**

Methods of isolating highly sialylated recombinant vitamin K dependent proteins, particularly Factor IX, by chromatographic methods are described. The highly sialylated recombinant proteins are characterized. The improved Factor IX has at least 62% N-glycosyslation with 3 or 4 sialic acid residues and improved bioavailability and pharmokinetic properties.

## Description

### RELATED APPLICATIONS

This application claims priority to U.S. Provisional Application No. 60/917,271, filed May 10, 2007 and U.S. Provisional Application No. 60/914,281, filed April 26, 2007. Both applications are incorporated herein by reference.

### BACKGROUND OF THE INVENTION

### Field of the Invention

Embodiments of the invention relate to production of recombinant Factor IX and variants, and other vitamin K dependent (VKD) proteins and variants with increased bioavailability. These VKD proteins are characterized by high sialic acid content.

### Description of the Related Art

The pharmacokinetic properties of recombinant Factor IX (rFactor IX, Benefix®) do not compare well with the properties of human plasma-derived Factor IX (pdFactor IX, Mononine®) after i.v. bolus infusion in laboratory animal model systems and in humans- Due to the less favorable pharmacokinetic properties of rFactor IX, generally 20-30% higher doses of rFactor IX are required to achieve the same procoagulant activity level as pdFactor IX (White, et al. (April 1998) Seminars in Hematology vol. 35, no. 2 Suppl. 2: 33-38; Roth, et al. (December 15, 2001) Blood vol. 98 (13): 3600-3606). There are several differences between rFactor IX and pdFactor IX, primarily in the levels of sulfation of Tyr 155 and phosphorylation at Ser 158, and while it has not been rigorously shown why they behave differently in vivo (Bond, et al. (April 1998) Seminars in Hematology vol. 35 no. 2 Suppl 2), it has been postulated that the primary reason is because of the difference in TYR 155 sulfation between plasma-derived and recombinant Factor IX (BENEFIX®, Summary of Basis for Approval, Reference no. 96-1048). Recently, the inventors have discovered that by increasing the level of N-glycan sialylation of rFactor IX the therapeutic potency of the laboratory preparations, as measured by increased bioavailability after i.v. bolus infusion into animal model systems, may be increasingly improved to achieve levels that exceed those previously reported for commercially available rFactor IX preparations while not significantly affecting other structural properties, e.g. Tyr 155 sulfation, of the protein.

One of the primary differences between Benefix® and Mononine®, is the oligosaccharide structures associated with the N-linked glycans that occur at ASN 157 and ASN 167. Mononine® contains N-glycans consisting almost entirely tri- and tetra-antennary oligosaccharides whereas Benefix® has primarily bi- and tri-antennary oligosaccharide structures with a small amount of tetra-antennary structures. These differences are not unexpected insofar as BeneFix is synthesized in a non-human mammalian cell with known differences in post-translation protein glycosylation. At the point of this invention there was no correlation, known to us, of the relationship between rFactor IX glycosylation and bioavailability. However, it was clear that relative to Mononine, only about 70% of i.v. infused Benefix® is recovered in patients resulting in a lower therapeutic potency and a requirement for a higher dosing regimen in order to control spontaneous bleeding.

The present inventors decided to correlate the extent and type of N-glycan modification of tissue culture produced rFactor IX with its recovery in mice, rats and eventually dogs- If we could identify that N-glycan structure and composition changes can lead to improved recovery or increased circulating half-life, a clinically and commercially superior rFactor IX product could then be synthesized. Clearly, if a Factor IX molecule could be synthesized which demonstrated better bioavailability in animals and/or longer circulating half-life, the therapeutic potency would be greater such that less of this molecule would have to be administrated to patients per dose. Thus, the clinical application would be safer (less product needed to be infused) and cheaper (more of the infused product recovered) for the treated hemophiliac.

The invention relates to the production of Factor IX by recombinant DNA technology in a tissue culture system. Embodiments of the invention relate to methods of manufacturing rFactor IX which produce material similar to human plasma derived material such that it is provided into the blood circulation of hemophiliacs for the treatment and/or prevention of spontaneous and traumatic bleeding episodes. The problems to be solved are (1) the initial recovery of as much rFactor IX material as possible and, (2) the circulation of the rFactor IX at clinically significant levels for as long as possible in the bloodstream of the patient after administration. These problems are common to other recombinant proteins used to treat or prevent blood coagulation disorders such as Factor VIIa and Protein C. The invention more generally applies to these recombinant proteins and others such as Prothrombin, Factor X and Protein S, that share the property of requiring vitamin K, i.e. vitamin K dependent (VKD) proteins, for the synthesis of biologically active proteins.

### Definitions

The term "pharmacokinetic properties" has its usual and customary meaning and refers to the absorption, distribution, metabolism and excretion of the VKD protein. In order to have improved pharmokinetic properties according to the invention, one or more of absorption, distribution, metabolism and excretion of the VKD protein is improved relative to a reference VKD protein, normally the corresponding VKD protein found in human plasma.

The usual and customary meaning of "bioavailability" is the fraction or amount of an administered dose of biologically active drug that reaches the systemic circulation. In the context of embodiments of the present invention, the term "bioavailability" includes the usual and customary meaning but, in addition, is taken to have a broader meaning to include the extent to which the VKD protein is bioactive. In the case of Factor IX, for example, one measurement of "bioavailability" is the procoagulant activity of the VKD protein obtained in the circulation post-infusion.

"Posttranslational modification" has its usual and customary meaning and includes but is not limited to removal of leader sequence, γ-carboxylation of glutamic acid residues, β-hydroxylation of aspartic acid residues, N-linked glycosylation of asparagine residues, O-linked glycosylation of serine and/or threonine residues, sulfation of tyrosine residues, and phosphorylation of serine residues.

As used herein, "biological activity" is determined with reference to a standard derived from human plasma. For Factor IX, the standard is MONONINE® (ZLB Behring). The biological activity of the standard is taken to be 100%.

The term "processing factor" is a broad term which includes any protein, peptide, non-peptide cofactor, substrate or nucleic acid which promotes the formation of a functional vitamin K dependent protein. Examples of such processing factors include, but are not limited to, paired basic amino acid converting (or cleaving) enzyme (PACE), Vitamin K epoxide reductase (VKOR), and Vitamin K dependent γ-glutamyl carboxylase (VKGC).

### BRIEF DESCRIPTION OF THE DRAWING

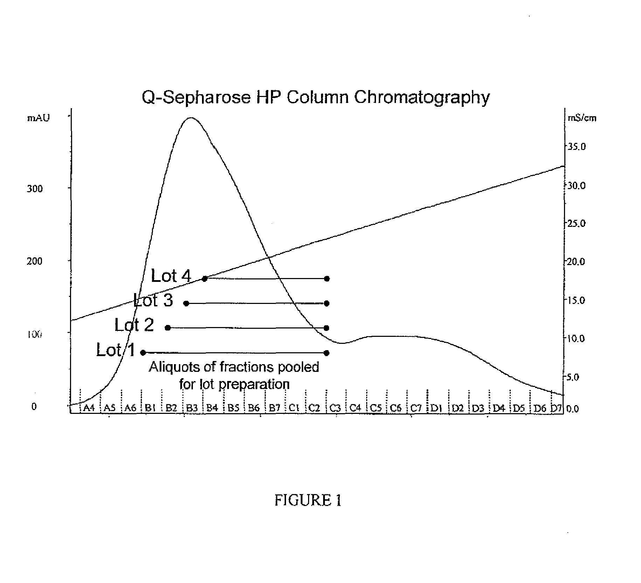

Figure 1 shows elution from a Q-Sepharose HP column which are described in Table 1.

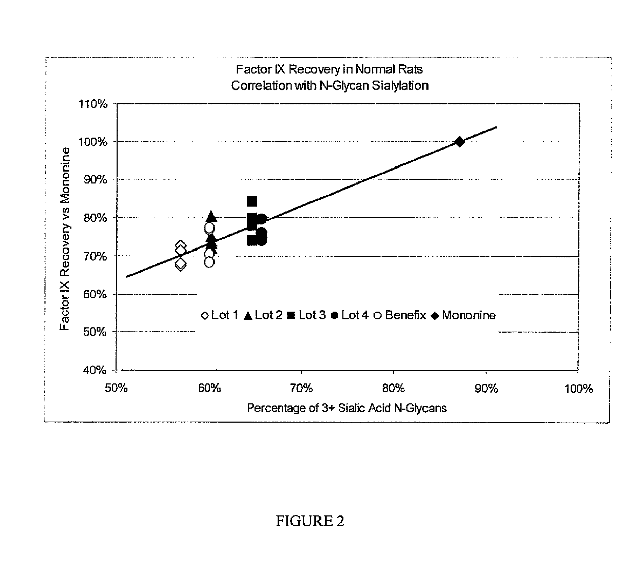

Figure 2 shows the percentage of N-glycosylation sites in recombinant Factor IX proteins which have 3 or more sialic acid residues correlated with Factor IX recovery determined by ELISA assay. Multiple lots of recombinant Factor IX containing varying levels of N-glycan sialylation (measured by using standardized methods for carbohydrate analysis) were infused intravenously at a standard dose (0.2 mg/kg) into normal rats. Plasma samples obtained at timed intervals post-infusion were analyzed for Factor IX protein by ELISA.

### DETAILED DESCRIPTION OF THE PREFERRED EMBODIMENT

Embodiments of the invention are directed to the production of recombinant VKD proteins, in particular Factor IX protein for the treatment of hemophilia, in high yield with improved bioavailability and bioactivity. Other VKD proteins include Factor II, Factor VII, Factor X, Protein C or Protein S. More preferably, the vitamin K dependent protein is Factor IX.

Factor IX is commercially available as both a plasma-derived product (Mononine®) and a recombinant protein (Benefix®). Mononine® has the disadvantage that there is a potential to transmit disease through contamination with bacteria and viruses (such as HIV, Hepatitis) which are carried through the purification procedure. The use of recombinant protein (Benefix®) avoids these problems. However, the bioavailability of Benefix® is poor compared to Mononine®. The goal is to provide the advantages of a recombinant protein with the high bioactivity of the isolated protein.

Factor IX protein in vivo undergoes extensive posttranslational modification including cleavage and removal of the pre-pro leader sequence of 46 amino acids, γ-carboxylation of the first 12 glutamic acid residues, partial β-hydroxylation of Asp 64, N-linked glycosylation of asparagines at positions 157 and 167. O-linked glycosylation at serine and threonine, and phosphorylation at serine 158. The cell lines used to produce recombinant Factor IX do not necessarily carry out all of these posttranslational modifications and it is not practical to optimize conditions to provide all of these modifications and also obtain a good yield of the recombinant protein. The present inventors have found that optimization of the N-glycosylation of Factor IX provides an improvement in functioning and bioavailability of Factor IX protein that was unexpected.

The scientific community has not been able to synthesize a Factor IX molecule in tissue culture, which reflects the structure of the human plasma-derived molecule. As a consequence it is not unexpected that the commercially available rFactor IX does not behave the same way as the plasma-derived protein when infused into hemophiliacs to treat disease. By comparison to pdFactor IX the primary problem is that 30% to 50% (Mononine Comparison Study Group, Transfusion 2002, 424:1-8) more of the injected rFactor IX is immediately cleared from the circulation. The result poses two problems for the hemophiliac. First, they need to receive more rFactor IX than they need for an effective therapeutic dose and are exposed to higher protein levels which raises safety issues (immunogenicity, etc.). Secondly, the cost of effective treatment with rFactor IX is increased by 50% to 100% because of the immediate loss ofrFactor IX from the circulation after i.v. infusion.

An advantage of this invention is that the bioavailability rFactor IX approximates the bioavailability of pdFactor IX. The rFactor IX molecule of the invention will have several features which will make it a clinically superior product for the treatment of Hemophilia B. First, compared with Benefix®, it allows more of the injected Factor IX to be recovered, requiring less of the exogenous and contaminating proteins to be exposed to the patient. This is a clear benefit to the patient in potential adverse event situations like thrombosis induction and inhibitor antibody formation. Secondly, when the new rFactor IX is infused into the patient, a significantly larger amount of the Factor IX will circulate for a longer time in the patient. Such a state leads to fewer infusions in either 'on demand' or prophylaxis treatment of hemophiliacs. Fewer infusions to control hemostasis in Hemophilia B patients is clearly a clinical advantage for the patient.

Producing rFactor IX in a tissue cell type system having clinical properties (1) better than Benefix® and (2) closer to Mononine® are goals of this invention. It is our belief that a rFactor IX with these properties will essentially replace Benefix® commercially for safety, efficacy and/or cost reasons.

Media/fermentation conditions have been screened to find ones that produce more highly sialylated Factor IX. Screening media/fermentation conditions to achieve a product of a given quality is well known and routine to one skilled in the art. Alternatively a preparation enriched in more highly sialylated Factor IX may be obtained by purification of the recombinant product to enrich in a Factor IX species that has the desired sialylation. In preferred embodiments, a Factor IX is obtained in which at least 60% of the N-glycosylation sites contain 3 or 4 sialic acid. More preferably, a Factor IX is obtained in which at least 62% of the N-glycosylation sites contain 3 or 4 sialic acid. Yet more preferably, a Factor IX is obtained in which at least 65% of the N-glycosylation sites contain 3 or 4 sialic acid. Yet more preferably, a Factor IX is obtained in which at least 70% of the N-glycosylation sites contain 3 or 4 sialic acid. Yet more preferably, a Factor IX is obtained in which at least 75% of the N-glycosylation sites contain 3 or 4 sialic acid. Yet more preferably, a Factor IX is obtained in which at least 85% of the N-glycosylation sites contain 3 or 4 sialic acid. Yet more preferably, a Factor IX is obtained in which at least 95% of the N-glycosylation sites contain 3 or 4 sialic acid. Most preferably, a Factor IX is obtained in which 100% of the N-glycosylation sites contain 3 or 4 sialic acid.

In preferred embodiments, a recombinant Factor IX protein is produced by one or more of the method steps described herein. More preferably, the recombinant Factor IX protein produced by the methods described is included in a pharmaceutical composition. Some preferred embodiments are directed to a kit which includes the recombinant Factor IX protein produced according to the methods described herein. Preferably, the recombinant Factor IX protein is used in a method of treating hemophilia by administering an effective amount of the recombinant Factor IX protein to a patient in need thereof.

Many expression vectors can be used to create genetically engineered cells. Some expression vectors are designed to express large quantities of recombinant proteins after amplification of transfected cells under a variety of conditions that favor selected, high expressing, cells. Some expression vectors are designed to express large quantities of recombinant proteins without the need for amplification under selection pressure. The present invention is not dependent on the use of any specific expression vector.

To create a genetically engineered cell to produce large quantities of a given vitamin K-dependent protein, cells are transfected with an expression vector that contains the cDNA encoding the protein. In some embodiments, the target protein is expressed with selected co-transfected enzymes that cause proper post-translational modification of the target protein to occur in a given Cell system.

In some embodiments, selected enzymes are co-transfected along with the vitamin K-dependent protein. For example, co-expression of an enzyme (PACE), facilitates removal of the propeptide region from vitamin K-dependent proteins.

In some embodiments, the method of the present invention involves the first selection of a cell that may be genetically engineered to produce large quantities of a vitamin K-dependent protein such as Factor IX.

The cell may be selected from a variety of sources, but is otherwise a cell that may be transfected with an expression vector containing a nucleic acid, preferably a cDNA of a vitamin K-dependent protein.

From a pool of transfected cells, clones are selected that produce quantities of the vitamin K-dependent protein over a range (Target Range) that extends from the highest level to the lowest level that is minimally acceptable for the production of a commercial product. Cell clones that produce quantities of the vitamin K-dependent protein within the Target Range may be combined to obtain a single pool or multiple sub-pools that divide the clones into populations of clones that produce high, medium or low levels of the vitamin K-dependent protein within the Target Range.

In some embodiments, deficiencies in post-translational modification of the vitamin K-dependent protein may be addressed by the simultaneous or subsequent (sequential) transfection of the cell clones with additional expression vectors containing cDNA for given proteins.

In some embodiments, the host cell may first be transfected with gene(s) encoding one or more processing factors and subsequently transfected with a gene encoding a vitamin K dependent protein. In some embodiments, the host cell is first transfected with a gene encoding a vitamin K dependent protein and subsequently transfected with one or more processing factors. Optionally, the host cell may be transfected with the gene(s) for the processing factor(s) or with the gene for the vitamin K dependent protein that is the same or substantially the same as an earlier transgene. After each round of transfection, clones are selected which express optimal levels of the transgene.

In some preferred embodiments, one such protein would have the enzymatic activity of vitamin K epoxide reductase (VKOR). In some preferred embodiments, another such enzyme would have the enzymatic activity of vitamin K-dependent gamma-glutamyl carboxylase (VKGC). In some preferred embodiments, another such enzyme would have the enzymatic activity of paired amino acid cleaving enzyme, i.e. PACE or furin. In some preferred embodiments, such enzymes would have glycosylation activity.

In some embodiments of the present invention, pools of cell clones that produce a vitamin K-dependent protein within the Target Range are subsequently transfected to provide a specific protein or multiple proteins in various combinations.

The practice of the present invention employs, unless otherwise indicated, conventional techniques of molecular biology, microbiology, recombinant DNA, and immunology, which are within the skill of the art. Such techniques are explained fully in the literature. See, e.g., Sambrook, et al., "Molecular Cloning; A Laboratory Manual", 2nd ed (1989); "DNA Cloning", Vols. I and II (D. N Glover ed. 1985); "Oligonucleotide Synthesis" (M. J. Gait ed. 1984); "Nucleic Acid Hybridization" (B. D. Hames & S. J. Higgins eds. 1984); "Transcription and Translation" (B. D. Hames & S. J. Higgins eds. 1984); "Animal Cell Culture" (R. I. Freshney ed. 1986); "Immobilized Cells and Enzymes" (IRL Press, 1986); B. Perbal, "A Practical Guide to Molecular Cloning" (1984); the series, Methods in Enzymology (Academic Press, Inc.), particularly Vols. 154 and 155 (Wu and Grossman, and Wu, eds., respectively); "Gene Transfer Vectors for Mammalian Cells" (J. H. Miller and M. P. Calos eds. 1987, Cold Spring Harbor Laboratory); " Immunochemical Methods in Cell and Molecular Biology", Mayer and Walker, eds. (Academic Press, London, 1987); Scopes, "Protein Purification: Principles and Practice", 2nd ed. 1987 (Springer-Verlag, N.Y.); and "Handbook of Experimental Immunology" Vols I-IV (D. M. Weir and C. C. Blackwell eds 1986). All patents, patent applications, and publications cited in the background and specification are incorporated herein by reference.

### Modification of the propeptide

In some embodiments, γ-carboxylation is increased by replacing the native propeptide sequence with a propeptide sequence that has a lower affinity for the gamma carboxylase as discussed in U.S. Application No. 2003/0220247, which is incorporated herein by reference. Useful propeptide sequences include altered forms of wild type sequences or propeptide sequences, or combinations of the same, for heterologous vitamin K dependent proteins. The propeptide sequence in vitamin K-dependent proteins is the recognition element for the enzyme which directs gamma carboxylation of the protein. Vitamin K-dependent proteins are not fully functional unless they comprise a high percentage of gamma carboxylated moieties. Thus, it is important when generating recombinant versions of these proteins that mechanisms be put in place to ensure full gamma carboxylation of the same.

The sequence alignment of several propeptide sequences is shown in FIG. 3 of US. 2003/0220247. Thus, propeptides which are useful in the present invention are those which have the sequences shown in FIG. 3 wherein an 18 amino acid sequence of several useful propeptides is shown along with the relative affinities of these propeptides for gamma carboxylase. A low affinity propeptide may be generated by modifying any one of amino acids - 9 or -13 on either prothrombin or protein C. Preferred modifications include the substitution of an Arg or a His residue at position -9 and the substitution of a Pro or a Ser residue at position - 13. Other preferred chimeric proteins include a propeptide selected from the group consisting of altered Factor IX, Factor X, Factor VII, Protein S, Protein C and prothrombin, or an unaltered propeptide in combination with the mature vitamin K dependent protein which is not native to the chosen propeptide sequence.

The term "fully gamma carboxylated protein" is used herein to refer to a protein wherein at least about 80% of the amino acids which should be gamma carboxylated are carboxylated. Preferably, at least about 85%, more preferably, at least about 90%, more preferably at least about 95% and even more preferably, at least about 99% of the amino acids which should be gamma carboxylated are gamma carboxylated.

### Paired basic amino acid converting enzyme (PACE)

As used herein, the term "PACE" is an acronym for paired basic amino acid converting (or cleaving) enzyme. PACE, originally isolated from a human liver cell line, is a subtilisin-like endopeptidase, i.e., a propeptide-cleaving enzyme which exhibits specificity for cleavage at basic residues of a polypeptide, e.g., -Lys-Arg-, -Arg-Arg, or -Lys-Lys-. PACE is stimulated by calcium ions; and inhibited by phenylmethyl sulfonyl fluoride (PMSF). A DNA sequence encoding PACE (or furin) appears in FIG. 1 [SEQ ID NO: 1] of U.S. Patent No. 5,460,950, which is incorporated herein by reference. The co-expression of PACE and a proprotein which requires processing for production of the mature protein results in high level expression of the mature protein. Additionally, co-expression of PACE with proteins requiring γ-carboxylation for biological activity permits the expression of increased yields of functional, biologically active mature proteins in eukaryotic, preferably mammalian, cells.

### Vitamin K dependent epoxide reductase

Vitamin K dependent epoxide reductase (VKOR) is important for vitamin K dependent proteins because vitamin K is converted to vitamin K epoxide during reactions in which it is a cofactor. The amount of vitamin K in the human diet is limited. Therefore, vitamin K epoxide must be converted back to vitamin K by VKOR to prevent depletion. VKOR sequences are known and available (see for example accession no. AY521634, Li, et al. ((2004) Nature 427: 541-544)- Consequently, co-transfection with VKOR provides sufficient vitamin K for proper functioning of the vitamin K dependent enzymes such as the vitamin K dependent γ-glutamyl carboxylase (VKCG). VKCG catalyzes γ-carboxylation of the gla-domain of vitamin K dependent coagulation factors.

### Vitamin K dependent gamma carboxylase

Vitamin K dependent γ-glutamyl carboxylase (VKGC) is an ER enzyme involved in the post-translation modification of vitamin K dependent proteins. VKGC incorporates CO₂ into glutamic acid to modify multiple residues within the vitamin K dependent protein within about 40 residues of the propeptide. The loss of three carboxylations markedly decreases the activity of vitamin K-dependent proteins such as vitamin K dependent coagulation factors. The cDNA sequence for human vitamin K dependent γ-glutamyl carboxylase is described by U.S. Patent No- 5,268,275, which is incorporated herein by reference. The sequence is provided in SEQ ID NO: 15 of U.S. Patent No. 5,268,275.

### Genetic Engineering Techniques

The production of cloned genes, recombinant DNA, vectors, transformed host cells, proteins and protein fragments by genetic engineering is well known. See, e.g., U.S. Pat. No. 4,761,371 to Bell et al. at Col. 6 line 3 to Col. 9 line 65; U.S. Pat. No. 4,877,729 to Clark et al. at Col. 4 line 38 to Col. 7 line 6; U.S. Pat. No. 4,912,038 to Schilling at Col. 3 line 26 to Col. 14 line 12; and U.S. Pat. No. 4,879,224 to Wallner at Col. 6 line 8 to Col. 8 line 59.

A vector is a replicable DNA construct. Vectors are used herein either to amplify DNA encoding Vitamin K Dependent Proteins and/or to express DNA which encodes Vitamin K Dependent Proteins. An expression vector is a replicable DNA construct in which a DNA sequence encoding a Vitamin K dependent protein is operably linked to suitable control sequences capable of effecting the expression of a Vitamin K dependent protein in a suitable host. The need for such control sequences will vary depending upon the host selected and the transformation method chosen. Generally, control sequences include a transcriptional promoter, an optional operator sequence to control transcription, a sequence encoding suitable mRNA ribosomal binding sites, and sequences which control the termination of transcription and translation.

Amplification vectors do not require expression control domains. All that is needed is the ability to replicate in a host, usually conferred by an origin of replication, and a selection gene to facilitate recognition of transformants.

Vectors comprise plasmids, viruses (e.g., adenovirus, cytomegalovirus), phage, and integratable DNA fragments (i.e., fragments integratable into the host genome by recombination). The vector replicates and functions independently of the host genome, or may, in some instances, integrate into the genome itself. Expression vectors should contain a promoter and RNA binding sites which are operably linked to the gene to be expressed and are operable in the host organism.

DNA regions are operably linked or operably associated when they are functionally related to each other. For example, a promoter is operably linked to a coding sequence if it controls the transcription of the sequence; or a ribosome binding site is operably linked to a coding sequence if it is positioned so as to permit translation.

Transformed host cells are cells which have been transformed or transfected with one or more Vitamin K dependent protein vector(s) constructed using recombinant DNA techniques.

### Host cells

Suitable host cells include prokaryote, yeast or higher eukaryotic cells such as mammalian cells and insect cells. Cells derived from multicellular organisms are a particularly suitable host for recombinant Vitamin K Dependent protein synthesis, and mammalian cells are particularly preferred. Propagation of such cells in cell culture has become a routine procedure (Tissue Culture, Academic Press, Kruse and Patterson, editors (1973)). Examples of useful host cell lines are VERO and HeLa cells, Chinese hamster ovary (CHO) cell lines, and W1138, HEK 293, BHK, COS-7, CV, and MDCK cell lines. Expression vectors for such cells ordinarily include (if necessary) an origin of replication, a promoter located upstream from the DNA encoding vitamin K dependent protein(s) to be expressed and operatively associated therewith, along with a ribosome binding site, an RNA splice site (if intron-containing genomic DNA is used), a polyadenylation site, and a transcriptional termination sequence. In a preferred embodiment, expression is carried out in Chinese Hamster Ovary (CHO) cells using the expression system of U.S. Patent No. 5,888,809, which is incorporated herein by reference.

The transcriptional and translational control sequences in expression vectors to be used in transforming vertebrate cells are often provided by viral sources. For example, commonly used promoters are derived from polyoma, Adenovirus 2, and Simian Virus 40 (SV40). See. e.g.. U.S. Pat. No. 4,599,308.

An origin of replication may be provided either by construction of the vector to include an exogenous origin, such as may be derived from SV 40 or other viral (e.g. Polyoma, Adenovirus, VSV, or BPV) source, or may be provided by the host cell chromosomal replication mechanism. If the vector is integrated into the host cell chromosome, the latter is often sufficient.

Rather than using vectors which contain viral origins of replication, one can transform mammalian cells by the method of cotransformation with a selectable marker and the DNA for the Vitamin K Dependent protein(s). Examples of suitable selectable markers are dihydrofolate reductase (DHFR) or thymidine kinase. This method is further described in U.S. Pat. No- 4,399,216 which is incorporated by reference.

Other methods suitable for adaptation to the synthesis of Vitamin K Dependent protein(s) in recombinant vertebrate cell culture include those described in M-J. Gething et al., Nature 293, 620 (1981); N. Mantei et al., Nature 281, 40; A. Levinson et al., EPO Application Nos. 117,060A and 117,058A.

Host cells such as insect cells (e.g., cultured Spodoptera frugiperda cells) and expression vectors such as the baculovirus expression vector (e.g., vectors derived from Autographa californica MNPV, Trichoplusia ni MNPV, Rachiplusia ou MNPV, or Galleria ou MNPV) may be employed in carrying out the present invention, as described in U.S. Pat. Nos. 4,745,051 and 4,879,236 to Smith et al. In general, a baculovirus expression vector comprises a baculovirus genome containing the gene to be expressed inserted into the polyhedrin gene at a position ranging from the polyhedrin transcriptional start signal to the ATG start site and under the transcriptional control of a baculovirus polyhedrin promoter.

Prokaryote host cells include gram negative or gram positive organisms, for example Escherichia coli (E. coli) or Bacilli. Higher eukaryotic cells include established cell lines of mammalian origin as described below. Exemplary host cells are E. coli W3110 (ATCC 27,325), E. coli B, E. coli X1776 (ATCC 31,537), E. coli 294 (ATCC 31,446). A broad variety of suitable prokaryotic and microbial vectors are available. E. coli is typically transformed using pBR322. Promoters most commonly used in recombinant microbial expression vectors include the betalactamase (penicillinase) and lactose promoter systems (Chang et al., Nature 275, 615 (1978); and Goeddel et al., Nature 281, 544 (1979)), a tryptophan (trp) promoter system (Goeddel et al., Nucleic Acids Res. 8,4057 (1980) and EPO App. Publ. No. 36,776) and the tac promoter (H. De Boer et al., Proc. Natl. Acad. Sci. USA 80, 21 (1983)). The promoter and Shine-Dalgarno sequence (for prokaryotic host expression) are operably linked to the DNA encoding the Vitamin K Dependent protein(s), i.e., they are positioned so as to promote transcription of Vitamin K Dependent Protein(s) messenger RNA from the DNA.

Eukaryotic microbes such as yeast cultures may also be transformed with Vitamin K Dependent Protein-encoding vectors. see, e.g., U.S. Pat. No. 4,745,057. Saccharomyces cerevisiae is the most commonly used among lower eukaryotic host microorganisms, although a number of other strains are commonly available. Yeast vectors may contain an origin of replication from the 2 micron yeast plasmid or an autonomously replicating sequence (ARS), a promoter, DNA encoding one or more Vitamin K Dependent proteins, sequences for polyadenylation and transcription termination, and a selection gene. An exemplary plasmid is YRp7, (Stinchcomb et al., Nature 282, 39 (1979); Kingsman et al., Gene 7, 141 (1979); Tschemper et al., Gene 10, 157 (1980)). Suitable promoting sequences in yeast vectors include the promoters for metallothionein, 3-phosphoglycerate kinase (Hitzeman et al., J. Biol. Chem. 255, 2073 (1980) or other glycolytic enzymes (Hess et al., J. Adv. Enzyme Reg. 7, 149 (1968); and Holland et al., Biochemistry 17, 4900 (1978)). Suitable vectors and promoters for use in yeast expression are further described in R. Hitzeman et al., EPO Publn. No. 73,657.

Cloned genes of the present invention may code for any species of origin, including mouse, rat, rabbit, cat, porcine, and human, but preferably code for Vitamin K dependent proteins of human origin. DNA encoding Vitamin K dependent proteins that is hybridizable with DNA encoding for proteins disclosed herein is also encompassed. Hybridization of such sequences may be carried out under conditions of reduced stringency or even stringent conditions (e.g., conditions represented by a wash stringency of 0,3M NaCl, 0.03M sodium citrate, 0.1% SDS at 60° C or even 70° C to DNA encoding the vitamin K dependent protein disclosed herein in a standard in situ hybridization assay. See J. Sambrook et al., Molecular Cloning, A Laboratory Manual (2d Ed. 1989)(Cold Spring Harbor Laboratory)).

As noted above, preferred embodiments of the present invention provide methods of producing recombinant Vitamin K dependent proteins by culturing recombinant cells under conditions which promote N-glycosylation and, optionally, include carboxylation of the N-terminal glu residues. This strategy may include co-expressing Vitamin K dependent protein along with VKOR, VKGC and/or PACE in a single host cell. In general, the method comprises culturing a host cell which expresses a vitamin K dependent protein and supporting proteins; and then harvesting the proteins from the culture. The culture can be carried out in any suitable fermentation vessel, with a growth media and under conditions appropriate for the expression of the vitamin K dependent protein(s) by the particular host cell chosen. In preferred embodiments, vitamin K dependent protein can be collected directly from the culture media, or the host cells lysed and the vitamin K dependent protein collected therefrom. In preferred embodiments, vitamin K dependent protein can then be further purified in accordance with known techniques.

As a general proposition, the purity of the recombinant protein produced according to the present invention will preferably be an appropriate purity known to the skilled art worker to lead to the optimal activity and stability of the protein. For example, when the recombinant protein is Factor IX , the Factor IX is preferably of ultrahigh purity. Preferably, the recombinant protein has been subjected to multiple chromatographic purification steps, such as affinity chromatography, ion-exchange chromatography and preferably immunoaffinity chromatography to remove substances which cause fragmentation, activation and/or degradation of the recombinant protein during manufacture, storage and/or use. Illustrative examples of such substances that are preferably removed by purification include thrombin and Factor IXa; other protein contaminants, such as modification enzymes like PACE/furin, VKOR, and VKGC; proteins, such as hamster proteins, which are released into the tissue culture media from the production cells during recombinant protein production; non-protein contaminants, such as lipids; and mixtures of protein and non-protein contaminants, such as lipoproteins. Purification procedures for vitamin K dependent proteins are known in the art. For example, see U.S. Patent No. 5,714,583, which is incorporated herein by reference. In preferred embodiments, the separation is done by conventional chromatography in the presence of calcium ions as described in US Patent No. 4,981,952 which is incorporated herein by reference. Calcium is generally present as a metal salt in the range of 5 to 50 mM, preferably 5 to 20 mM. Preferably calcium is in the form of calcium chloride, although other forms of calcium such as calcium acetate may be used.

In some embodiments, the VKD protein preparation is further fractionated on the basis of its glycosylation pattern. In preferred embodiments, sialylated mono-, di-, tri- and tetra- antennary VKD proteins are separated, preferably by chromatographic methods.

Factor IX DNA coding sequences, along with vectors and host cells for the expression thereof, are disclosed in European Patent App. 373012, European Patent App. 251874, PCT Patent Appl. 8505376, PCT Patent Appln. 8505125, European Patent Appln. 162782, and PCT Patent Appln. 8400560. Genes for other coagulation factors are also known and available, for example, Factor II (Accession No- NM_000506), Factor VII (Accession No. NM_019616, and Factor X (Accession No. NM_000504).

### EXAMPLES

### Example 1. Sialic acid profiling of rFactor IX preparations

Transfected CHO cells were grown in a 15 L bioreactor for 12 days in a fed batch production mode to obtain approximately 10 L of conditioned media containing rFactor IX. After harvest, the conditioned media was clarified to remove unwanted cells and cell debris and concentrated prior to protein purification. Protein purification was performed using pseudo-affinity column chromatography methods designed to separate forms of rFactor IX that bind calcium ions from forms that cannot (Yan 1991 Pat No. 4,981,952).

Recombinant Factor IX (rFactor IX) was fractionated by salt gradient elution of rFactor IX bound to Q-Sepharose HP in the presence of calcium (Figure 1). In this example, a Q-Sepharose HP chromatography column was prepared and equilibrated with a buffer solution containing 20 mM Bis-Tris, pH 6.0 and 10 mM calcium chloride. A solution of similar composition, but containing rFactor was applied to the column to adsorb Factor IX. After washing the column with equilibration buffer, protein was eluted by applying a salt gradient from 0 to 0.4 M sodium chloride. Column fractions were collected and absorbance at 280nm monitored to detect protein concentration.

Samples from selected fractions were digested with PNGase F to release N-linked oligosaccharides for analysis. The relative percentages of the sialylated N-glycans present in fractions identified in Figure 1 is shown below in Table 1. As can be seen from Table 1, the fractions differ in the composition of N-glycans with more rFactor IX having a higher percentage of N-glycans containing 3 or more sialic acids eluting from the column at higher salt concentration. By this means, fractions enriched in tri- and tetra-antennary Factor IX were identified.

**TABLE 1. Tabular presentation of the percentage for each group of N-glycans (based on sialic acid content) for the rFIX samples. All samples were digested with peptide N-glycosidase F (PNGaseF) in duplicate, and the released N-glycans were labeled for detection and analyzed by HPLC (Anumula and Dhume (1998) Glycobiology 8:685-694).**

| N-Glycan Composition | Q-Sepharose HP Column Fractions | | | | | | | | |
|---|---|---|---|---|---|---|---|---|---|
| | B1 | 82 | B3 | B4 | B5 | B6 | B7 | C1 | C2 |
| Neutral Glycans | 2% | 1% | 0% | 0% | 0% | 0% | 0% | 0% | 1% |
| 1 Sialic Acid | 12% | 10% | 4% | 5% | 4% | 3% | 3% | 4% | 3% |
| 2 Sialic Acids | 58% | 50% | 35% | 34% | 32% | 28% | 27% | 27% | 26% |
| 3 Sialic Acids | 29% | 37% | 58% | 50% | 50% | 57% | 56% | 55% | 52% |
| 4 Sialic Acids | 0% | 1% | 4% | 11% | 13% | 12% | 13% | 14% | 18% |

### Example 2. Highly Sialylated rFactor IX Preparations

To obtain preparations of highly sialylated rFactor IX for treating hemophilia, conditioned media obtained by cell culture methods were subjected to protein purification whereby one or more chromatographic steps are performed under pseudo-affinity conditions to separate fully gamma-carboxylated forms of Factor IX from under-carboxylated forms (Yan 1991 Pat No. 4,981,952). Fully gamma-carboxylated forms of Factor IX were further fractionated by column chromatography to obtain fractions containing increasing amounts (relative percentages) of protein with 3 or more sialic acid residues per N-glycan (Example 1). To obtain preparations of rFactor IX having a reasonable percentage of protein with 3 or more sialic acid residues, essentially all fractions may be pooled. To obtain preparations of rFactor IX having the greatest percentage of protein with 3 or more sialic acid residues per N-glycan, fractions eluting later from the column may be pooled. In general, the composition of rFactor IX with respect to sialic acid content in a given preparation may be adjusted to achieve a given target range as illustrated in Table 2.

**TABLE 2**

| Factor IX Preparation (Fractions Pooled) | Functional Protein Yield | Composition 3+SA N-Glycan |
|---|---|---|
| B1-C2 | 100% | 57% |
| B2-C2 | 90% | 60% |
| B3-C2 | 74% | 65% |
| B4-C2 | 57% | 66% |
| B5-C2 | 42% | 67% |
| B6-C2 | 29% | 69% |
| B7-C2 | 18% | 70% |
| C1-C2 | 10% | 70% |
| C2 | 5% | 70% |

Table 2 shows functional protein yield and 3+SA N-glycan content for pooled fractions from Table I.

### Example 3. Bioavailability of Highly Sialylated rFactor IX Preparations

Recombinant Factor IX preparations were obtained by pooling fractions shown in Figure 1 to obtain four unique lots (Lots 1-4) of Factor IX for in vivo analysis for bioavailability. The rFactor IX lots so produced varied in terms of the percentage of N-glycans that contained 3 or more (3+) sialic acid residues per glycan as shown in Table 3 .

**TABLE 3**

| Factor IX Preparation | 3+ SA N-Glycan | AUC | | Initial Recovery | | |
|---|---|---|---|---|---|---|
| | | 480 min | 1440 min | 2 min | 5 min | 15min |
| Lot 1 | 57% | 68% | 73% | 71% | 71% | 68% |
| Lot 2 | 60% | 73% | 80% | 74% | 75% | 72% |
| Lot 3 | 65% | 80% | 84% | 79% | 78% | 74% |
| Lot 4 | 66% | 74% | 80% | 80% | 76% | 74% |
| Mononine | 87% | 100% | 100% | 100% | 100% | 100% |
| Benefix | 60% | 70% | 77% | 77% | 70% | 68% |

For each rFactor IX lot and for preparations of BeneFix and Mononine, standardized dosing solutions were prepared and infused intravenously into normal Sprague-Dawley rats. At timed intervals after infusion plasma samples were collected to measure the amount of Factor IX antigen present in the circulation. The "initial" Factor IX recovery was defined as the amount of Factor IX antigen present in the circulation at 2, 5 and 15 minutes and the overall bioavailability was defined as the 'area under the curve' over 480 and 1440 minutes. Each rFactor IX preparation was evaluated in four (4) animals and the results averaged for comparison with results obtained pdFactor IX (Mononine) which were taken as 100%. This comparison is shown in Table 3. As shown in Figure 2, the initial recovery and bioavailability (AUC) of rFactor IX in normal rats is dependent on the percentage of N-glycans that contain 3 or more sialic acid residues. Preparations of rFactor IX having a lower percentage of 3+ sialic than BeneFix have a lower recovery and bioavailability whereas preparations having a high percentage have a higher recovery and bioavailability.

It will be understood by those of skill in the art that numerous and various modifications can be made without departing from the spirit of the present invention. Therefore, it should be clearly understood that the forms of the present invention are illustrative only and are not intended to limit the scope of the present invention.

### Items

1. A method of isolating highly sialylated Factor IX for treatment of hemophilia comprising;
   providing a preparation of Factor IX; and
   separating highly sialylated forms of Factor IX.
2. The method of item 1, wherein the separation is carried out by chromatography.
3. The method of item 2, wherein the chromatography is carried out in the presence of calcium.
4. The method of any one of items 1 to 3, wherein the Factor IX is fully gamma-carboxylated.
5. The method of any one of items 1-4, further comprising:
   collecting fractions enriched in highly sialylated Factor IX; and
   pooling the fractions to obtain a preparation having at least 50% N-glycans with 3 or more sialic acid residues.
6. The method of any one of items 1-5, wherein Factor IX is recombinant.
7. A recombinant vitamin K dependent (VKD) protein having pharmacokinetic properties that are comparable to or better than the pharmacokinetic properties of the corresponding vitamin K dependent protein derived from normal human plasma.
8. The recombinant VKD protein of item 7 wherein the VKD protein comprises N-linked oligosaccharides which are highly sialylated.
9. The recombinant VKD protein of item 8 wherein the percentage of N-linked oligosaccharides with 3 or more sialic acid residues per molecule is at least 62%.
10. The recombinant vitamin K dependent (VKD) protein of any one of items 7 to 9, wherein the VKD protein is selected from the group consisting of Factor VII, Factor IX, Factor X, Prothrombin and Protein C and structural variants of each having pharmacokinetic properties that are comparable to or better than the pharmacokinetic properties of the corresponding vitamin K dependent protein present in normal human plasma.
11. The recombinant VKD protein of any one of items 7 to 10 having ≥100% of the initial plasma recovery after intravenous infusion relative to the corresponding VKD protein derived from normal human plasma.
12. The recombinant VKD protein of any one of items 7 to 10 having >80% of the initial plasma recovery after intravenous infusion relative to the corresponding VKD protein derived from normal human plasma.
13. The recombinant VKD protein of any one of items 7-10 having ≥100% of the bioavailability (AUC) after intravenous infusion relative to the corresponding VKD protein derived from normal human plasma.
14. The recombinant VKD protein of any one of items 7-10 having >80% of the bioavailability (AUC) after intravenous infusion relative to the corresponding VKD protein derived from normal human plasma.
15. A method of improving the bioavailability of recombinant VKD proteins when administered to a patient in need thereof which comprises increasing the glycosylation of the recombinant VKD.
16. A preparation comprising a recombinant VKD protein which is free from contamination with plasma proteins other than the VKD protein, wherein the preparation has pharmacokinetc proterties that are comparable to or better than the pharmacokinetic properties of the corresponding VKD protein derived from normal human plasma.
17. The preparation of item 6, wherein the VKD protein comprises N-linked oligosaccharides which are highly sialylated.
18. The preparation of item 17, wherein the percentage of N-linked oligosaccharides with 3 or more sialic acid residues per molecule is at least 62%.
19. The preparation of any one of items 16 to 18, wherein the recombinant vitamin K dependent (VKD) blood coagulation protein is selected from the group consisting of Factor VII, Factor IX, Factor X, Prothrombin and Protein C and structural variants of each having pharmacokinetic properties that are comparable to or better than the pharmacokinetic properties of the corresponding vitamin K dependent protein present in normal human plasma.

## Claims

1. A recombinant Factor IX wherein the percent of N-linked glycans with 4 sialic acids per molecule is at least 11%.

2. The recombinant Factor IX of claim 1, wherein the percent of N-linked glycans with 4 sialic acids per molecule is at least 12%.

3. The recombinant Factor IX of claim 2, wherein the percent of N-linked glycans with 4 sialic acids per molecule is at least 13%.

4. The recombinant Factor IX of claim 3, wherein the percent of N-linked glycans with 4 sialic acids per molecule is at least 14%.

5. The recombinant Factor IX of claim 4, wherein the percent of N-linked glycans with 4 sialic acids per molecule is at least 18%.

6. A pharmaceutical composition comprising the recombinant Factor IX according to any one of claims 1 to 5 for use in a method for treating hemophilia.

7. A pharmaceutical composition comprising the preparation according to any one of claims 1 to 5 for use in a method for treating hemophilia.

8. A kit comprising the recombinant Factor IX according to any one of claims 1 to 5.

9. A kit comprising the preparation according to any one of claims 1 to 5.
